# EUROPEAN PATENT APPLICATION

(11) **EP 1 889 831 A2**
(43) Date of publication of application: **20.02.2008**
(21) Application number: 07468010.9
(22) Date of filing: 07.08.2007
(51) Int. Cl.: C07C 281/20, C07D 213/40, A61K 31/4402, A61K 31/4406, A61K 31/175, A61P 31/04

(54) **Diazenedicarboxamides as inhibitors of D-alanine: D-alanine ligase (Ddl)**

(30) Priority: 18.08.2006 SI 200600186
(71) Applicant: University of Ljubljana, 1000 Ljubljana (SI); University of Ljubljana, 1000 Ljubljana (SI); Antimicrobial Research Centre, Leeds LS2 9JT (GB)
(72) Inventor: Gobec, Stanislav, 1000 Ljubljana (SI); Kovac, Andreja, 9226 Moravske Toplice (SI); Brajic, Alja, 1000 Ljubljana (SI); Pecar, Slavko, 1235 Radomlje (SI); Bostock, Julieanne, Chapel Allerton, Leeds LS7 3NL (GB); Chopra, Ian, Pool in Wharfdale, Leeds LS21 1RR (GB); Lenarsic, Roman, 1000 Ljubljana (SI); Bombek, Sergeja, 8000 Novo Mesto (SI); Kocevar, Marijan, 1000 Ljubljana (SI); Polanc, Slovenko, 1230 Domzale (SI)

(57) **Abstract**

This invention belongs to the field of pharmaceutical chemistry and relates to new diazenedicarboxamides as inhibitors of D-alanine:D-alanine ligaze (Ddl), to procedures for their preparation and pharmaceutical preparations containing the same. Compounds of general formula I: 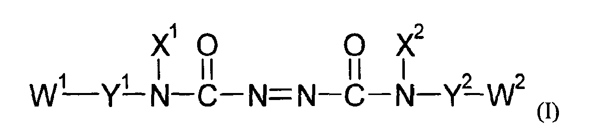 and the pharmaceutically acceptable salts are described. The appropriate substituents are clearly presented in the body of the text and in claims.

## Description

### Description of invention

This invention belongs to the field of pharmaceutical chemistry and relates to new diazenedicarboxamides as inhibitors of bacterial D-alanine:D-alanine ligase (Ddl), to procedures for their preparation and pharmaceutical preparations containing the same. New diazendicarboxamides are inhibitors of Ddl ligase, which is an enzyme involved in biosynthesis of bacterial peptidoglycan, and have antimicrobial activity.

### Technical problem

The emergence of resistant pathogenic bacteria to antimicrobials in use for treatment of infectious disease is increasing (Ritter, T.K.; Wong, C.-H. Angew. Chem. Int. Ed. 2001, 40, 3508-3533; Davies, J. Nature 1996, 383, 219-220; Levy, S.B. Sci. Am. 1998, 278, 46-53). Cerain strains of three bacterial species (*Enterococcus faecalis, Mycobacterium tuberculosis, Pseudomonas aeuginosa*) responsible for life-threatening conditions are already resistant to all antibiotics used in clinical therapy. To avoid this situation new antimicrobial agents directed towards previously unused targets need to be discovered.

### The state of technique

Peptidoglycan is an essential bacterial cell-wall polymer unique to prokaryotic cells and therefore optimal for the selective targeting of microbial vital pathways. The early steps of peptidoglycan biosynthesis that take place in the cytoplasm are relatively unexploited and therefore represent suitable targets for drug discovery. Amino acid ligases MurC, MurD and MurE catalize the addition of L-alanine (L-Ala), D-glutamic acid (D-Glu) and *meso-*diaminopimelic acid or L-lysine (L-Lys), respectively, to the growing UDP-*N*-acetylmuramoylpentapeptide, while MurF catalyzes the addition of dipeptide D-alanyl-D-alanine (van Heijenoort, J. Nat. Prod. Rep. 2001, 18, 503-519). Dipeptide D-alanyl-D-alanine is formed by the action of two enzymes: the first is alanine racemase which catalyzes the conversion of L-alanine into D-alanine and the second is Ddl ligase. Inhibitors of all abovementioned enzymes are potential antimicrobial agents.

Ddl ligase is the cytoplasmatic enzyme responsible for the formation of D-alanyl-D-alanine dipeptide according to the reaction: D-Ala + D-Ala + ATP ↔ D-Ala-D-Ala + ADP + Pi

The reaction mechanism of Ddl ligase proceeds via an acyl-phosphate intermediate and is similar to the mechanisms of other ATP-dependant ligases (like glutamine synthetase, glutathione synthetase, Mur ligases). The enzyme initially phosphorylates the free carboxylic acid of the first D-alanine with the γ-phosphate group of ATP so that the acyl-phosphate intermediate is formed. The activated carboxylic acid is then attacked by the amino group of the incoming second D-alanine. The reaction proceeds via a high-energy tetrahedral intermediate and ends by the release of the free phosphate and the peptide bond containing product. For proper activity, Ddl enzyme needs Mg²⁺ ions. They coordinate the binding of the substrates into the active site, balance the charge of the active site and consequently stabilize the complex between substrate and enzyme. An acyl-phosphate mechanism was also confirmed by crystallographic analysis of the enzyme-phosphorylated phosphinate inhibitor complex (Fan, C., Park, I.S., Walsh, C.T., Knox, J.R. Biochemistry 1997; 36: 2531-2538).

There are two isoenzymes of Ddl: DDlA and DDlB. Despite differences in their size (DDlB enzyme is smaller) and only 35% of amino acid homology, both enzymes have very similar catalytic efficiency and substrate specificity (Zawadzke, L. E., Burr, T. D. H., Walsh, C. T.: Biochemistry 1991; 30: 1673-1682). Sensitivity to inhibitors is similar for both isoforms as well.

The most important Ddl inhibitor is D-cycloserine, which is clinically used for the treatment of tuberculosis (Wargel, R. J., Shadur, C. A., Neuhaus, F. C.: J. Bacteriol. 1970; 103: 778-788). Beside Ddl, D-cycloserine inhibits also alanine racemase, which provides the substrate for Ddl. Several transition-state analogues, such as phosphinates, phosphonates and phosphonamidates are also known as inhibitors of Ddl. They were employed to confirm the acyl-phosphate reaction mechanism. After they become phosphorylated in the active site, transition-state analogues have a very strong resemblance to the natural transition-state intermediate and therefore they strongly inhibit Ddl (Chakravarty, P. K.,Greenlee, W. J., Parsons, W. H., Patchett, A. A., Combs, P., Roth, A., Busch, R. D., Mellin, T. N.: J. Med. Chem. 1989 32: 1886-1890; Busch, R. D., Mellin, T. N., Valiant, M. E., Weissberger, B., Gadebusch, H., Springer, J. P., Combs, P. L., Davidson, J., Taub, D., Schoen, W. R., Bull, H. G., Patchett, A. A., Parsons, W. H.: J. Med. Chem. 1988; 31: 1772-1778). Phosphinate and phosphonate dipeptide analogues of transition-state intermediates that inhibit both DdlA and DdlB were prepared by Bartlett and coworkers in 1996 (Tom, N. J., Ellsworth, B. A., Bartlett, P. A.: Chem. & Biol. 1996; 3: 37-44). Recently, a new cyclopropyl inhibitor of Ddl was developed using computer based *de novo* design (software SPROUT) (Lloyd, A. J., Roper, D. I., Chopra, I., Stubbings, W., Bostock, J. M., Besong, G. E., Fishwick, C. W. G., Johnson, P.: Angew. Chem. Int. Ed. 2005; 44: 2-6).

### Description of solution of the technical problem including examples

The invention relates to novel compounds with the general formula (I) 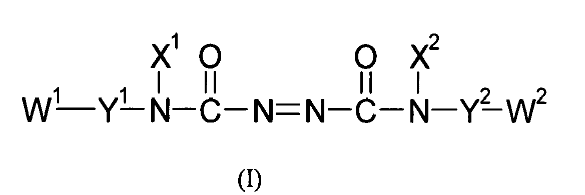 wherein:
**X¹, X²**: - H, C₁₋₆ linear or branched alkyl, (aryl) -NH- , -O-, -(CH₂)ₙ- (where n can be between 0 and 5), C₁₋₆ branched alkyl;
**Y¹, Y²**: -(CH₂)ₙ- (where n can be between 0 and 5), C₁₋₆ branched alkyl; -NH-, -C=O; -NHCO-, -CONH-;
**W¹, W²**: - H,
   - C₁₋₆ linear or branched alkyl. Alkyl can either be unsubstituted or substituted with one or more substituents, e.g., nitro, halogen, trifluoromethyl or hydroxyl,
   - phenyl, which can either be unsubtsituted or substituted with one or more substituents, e.g., nitro, halogen, trifluoromethyl, hydroxyl, C₁₋₈ linear or branched alkyl, C₁₋₈ linear or branched alkoxy, unsubstituted phenyl or substituted phenyl,
   - naphthyl, which can be either unsubsituted or substituted,
   - fluorenyl,
   - 5- to 7- membered monocyclic or 7- to 10-membered bicyclic heterocyclic ring system, which can be unsubstituted or substituted and can beside carbon atoms contain 3 or less heteroatoms such as N, O, P or S,
   - 3- to 10-membered monocyclic or 7- to 10- membered carbocyclic ring system, which can be unsubstituted or substituted and can beside carbon atoms contain 3 or less heteroatoms such as N, O, P or S.

Some compounds in this invention have one or more stereogenic centers, where absolute configuration can be R or S. They can be as racemates, conglomerates, pure enantiomers, mixtures of diastereoisomers or pure diastereoisomers.

The invention relates also to pharmaceutically acceptable salts of compounds with general formula I.

The invention relates to the use of compounds with general formula I as therapeutically active substances for drug preparation. New compounds are inhibitors of D-alnine:D-alanine ligase and can be used for treatment of bacterial infections.

The invention relates also to pharmaceutical compositions containing compounds with general formula I. They can be in forms of injectable preparations, peroral preparations, topical applications or other pharmaceutically suitable forms. Pharmaceutical forms are prepared according to standard procedures and can beside the active compound contain also proper auxiliary substances. They can be also incorporated in different dosage forms with modified release, e.g., with controlled or prolonged release. The dosage to be administered depends to a large extent upon the condition and size of the subject being treated as well as the route and frequency of administration. Such matters, however, are typically left to the discretion of the clinician according to principles of treatment well known in the antibacterial arts.

The invention relates also to procedures for preparation of compounds with general formula I.
Diazenedicarboxamides of general formula I can be prepared:
1. Unsymmetrical derivative is synthesized from isocyanate with formula II (prepared from the corresponding amine with phosgene, diphosgene or triphosgene in dry aprotic solvent in the presence of tertiary amine) and carbazate III (R = C₁₋₆ linear or branched alkyl) in aprotic solvent. 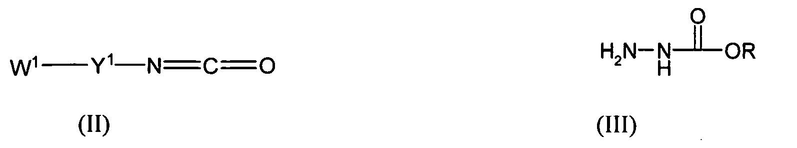 This results in the corresponding 1,4-disubstituted semicarbazide IV. The same compound can be prepared from 4-substituted semicarbazide V and chloroformate VI (R = C₁₋₆ linear or branched alkyl), if semicarbazide V reacts with chloroformate VI. 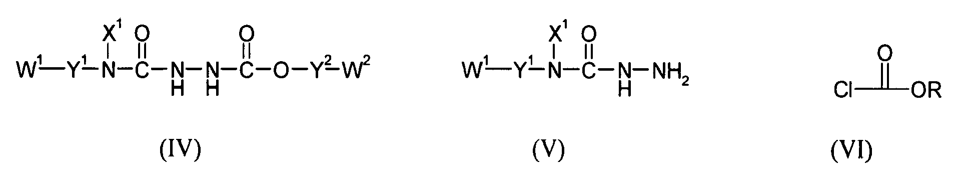 Resulted 1,4-disubstituted semicarbazide IV is transformed to aminocarbonyldiazenecarboxylate VII with suitable organic or inorganic oxidizing agent. Substitution of alkoxy group of aminocarbonyldiazenecarboxylate VII with primary or secondary amines VIII results in diazenedicarboxamide I. 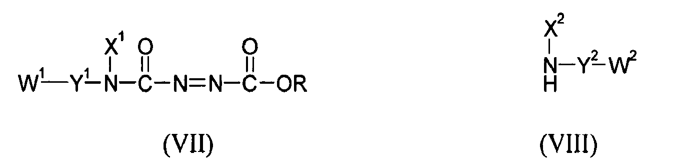
2. Symmetrical diazenedicarboxamide derivative I (X¹ = X², Y¹ = Y², W¹ = W²) can be prepared from diazenedicarboxylate and two or more equivalents of primary or secondary amine in protic or aprotic solvent. Nucleophilic substitution of both alkoxy groups can be also carried out in the absence of solvent.

### Biological data

### Determination of inhibitory activity on DdlB from E. coli

The D-Ala-adding activity of DdlB ligase was monitored by the detection of orthophosphate generated during the reaction based on the colorimetric malachite green method (Walsh, A. et al. J Bacteriol. 1999, 181: 5395). Assays were performed at 37 °C in a mixture (final volume: 50 µl) containing 38.5 mM Hepes, pH 8.0, 3.25 mM MgCl₂, 6.5 mM (NH₄)₂SO₄, 700 µM D-Ala, 500 µM ATP, purified DDIB (diluted in 20 mM Hepes, pH 7.2, and 1 mM dithiothreitol), and 500 µM test compound (IC₅₀ values were determined for a range of inhibitor concentrations). All compounds were soluble in the assay mixture containing 5% DMSO. After 30 min of incubation, 100 µL Biomol^{®} reagent was added. After 5 min, absorbance was read at 650 nm. Residual activity was calculated with respect to a similar assay without inhibitor. IC₅₀ constants were determined by measuring residual activity at seven different inhibitor concentrations and represent the concentration of inhibitors where RA is 50%.

### Determination of antimicrobial activity

Minimal inhibitory concentrations (MICs) of compounds were determined by broth microdilution in IsoSensitest broth (Oxoid, Basingstoke, UK) using an inoculum of 10⁴ cells per mL for *E. coli* or 10⁶ cells per mL for *S*. *aureus.* The compounds under investigation were prepared in a 2-fold dilution series in 50% dimethyl sulfoxide (Sigma-Aldrich, Dorset, UK). Microwell plates with 96 wells (Nunc, Fisher Scientific, Loughborough, UK) containing the antimicrobial agent and bacterial suspension were incubated for 16 h at 37 °C in a Spectramax 384 plus microwell plate reader (Molecular Devices, Abingdon, UK), running SOFTmax PRO 3.1.1 software. Optical density readings (600 nm) were taken at 10 min intervals. Plates were shaken for 30 s before each reading. The MIC was taken as the lowest concentration of antimicrobial agent that prevented growth.
The invention is further described in connection with the following non-limiting examples:

### EXAMPLE 1

### Synthesis of N¹-(4-Isopropylphenyl)-N²-(2-chloroethyl)-1,2-diazenedicarboxamide

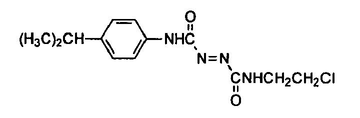

### Step 1

### Methyl (4-Isopropylanilino)carbonylhydrazinecarboxylate

A solution of 4-isopropylaniline (1.45 mL, 10 mmol) in dichloromethane (25 mL) was added dropwise to the stirred solution of triphosgene (1.10 g, 3.7 mmol) in dichloromethane (25 mL) under argon at rt. A suspension was cooled to 0 °C and triethylamine was added (3 mL, 23.3 mmol). After 5 min, a methyl hydrazinecarboxylate was added (900 mg, 10 mmol). The reaction mixture was stirred for 10 min at 0 °C, followed by stirring at rt for an additional 10 min. Then HCl was added (30 mL, 1M) and a white solid was filtered off, washed with water (10 mL) and cold diethyl ether (15 mL) to give the desired product (2.01 g, 80% yield).
mp = 169.5-172.3 °C (ethyl acetate)
IR (KBr) 3357, 3306, 3251, 2959, 1737, 1705, 1686, 1610, 1548, 1517, 1244 cm⁻¹.
¹H NMR (DMSO-*d*₆) δ 1.17 (d, *J =* 6.8 Hz, 6H), 2.81 (h, J = 6.8 Hz, 1H), 3.60 (s, 3H), 7.11 (m, 2H), 7.35 (m, 2H), 7.95 (broad s, 1H), 8.60 (s, 1H), 8.93 (broad s, 1H).
¹³C NMR (DMSO-*d*₆) δ 24.0, 32.7, 51.8, 118.6, 126.2, 137.3, 141.8, 155.6, 157.3.
MS (FAB) *m*/*z* (%) 252 (M⁺ + H, 70), 91 (100).
Anal. Calcd for C₁₂H₁₇N₃O₃ (M = 251.28 g/mol): C, 57.36; H, 6.82; N, 16.72. Found: C, 57.15; H, 7.02; N, 17.01.

### Step 2

### Methyl (4-Isopropylanilino)carbonyldiazenecarboxylate

Pyridine (1.13 mL, 14 mmol) was added to a stirred suspension of methyl (4-isopropylanilino)carbonylhydrazinecarboxylate (1.76 g, 7 mmol) in dichloromethane (20 mL) at rt. Then, NBS (1.37 g, 7.7 mmol) was slowly added to the suspension which was stirred for 1 h at rt and then treated with HCl solution (1 : 1, 10 mL). Two phases were separated and an organic one was washed with 5% aqueous solution of Na₂S₂O₃.5H₂O (15 mL), saturated NaHCO₃ (15 mL), and water (15 mL). An organic phase was dried over anhydrous Na₂SO₄ and the solvent was removed under reduced pressure to afford 1.70 g (98%) of the diazenedicarboxylate.
mp: oily product
IR (NaCl) 3271, 2961, 1773, 1732, 1602, 1559, 1519, 1437, 1417, 1251 cm⁻¹.
¹H NMR (DMSO-d₆) δ 1.20 (d, *J* = 7.2 Hz, 6H), (h, *J* = 7.2 Hz, 1H), 4.07 (s, 3H), 7.29 (m, 2H), 7.64 (m, 2H), 11.45 (s, 1 H).
^{l3}C NMR (DMSO-*d₆*) δ 23.8, 32.9, 55.6, 119.7, 126.8, 134.9, 145.4, 156.9, 161.9.
MS (FAB) m/z (%) 250 (M⁺ + H, 53), 154 (61), 91 (68), 69 (80), 55 (100).
Molecular formula: C₁₂H₁₅N₃O₃ M = 249.27 g/mol

### Step 3

### N²-(2-Chloroethyl)-N¹-(4-isopropylphenyl)- 1,2-diazenedicarboxamide

To a stirred suspension of methyl (4-isopropylanilino)carbonyldiazenecarboxylate (1.16 g, 4.65 mmol) and Na₂CO₃ (477 mg, 4.5 mmol) in dichloromethane (7 mL) 2-chloroethylamine hydrochloride (522 mg, 4.5 mmol) was added at 0 °C. After being stirred for 2 h at 0 °C, the reaction mixture was evaporated to dryness and the residue suspended in ethyl acetate (15 mL). An ethyl acetate solution was washed with water (2 × 10 mL), dried over anhydrous Na₂SO₄ and evaporated under reduced pressure to achieve 1.09 g (82%) of the final product.
mp = 148.5-150.4 °C (dichloromethane)
IR (KBr) 3245, 2962, 1737, 1709, 1536, 1415, 1255, 1062, 830 cm⁻¹.
¹H NMR (DMSO-d₆) δ 1.20 (d, *J* = 7.1 Hz, 6H), 2.88 (h, *J* = 7.1 Hz, 1H), 3.63 (m, 2H), 3.78 (t, *J* = 5.8 Hz, 2H), 7.28 (m, 2H), 7.64 (m, 2H), 9.23 (broad t, *J* = 5.6 Hz, 1 H), 11.27 (s, 1H).
¹³C NMR (DMSO-d₆) δ 23.8, 32.9, 42.1, 42.9, 119.7, 126.8, 135.2, 145.1, 158.1, 161.9.
MS (FAB) m/z (%) 299 ((M + 2)⁺ + H, 47), 194 (85), 138 (100), 120(61).
Anal. Calcd for C₁₃H₁₇ClN₄O₂ (M = 296.75 g/mol): 52.62% C, 5.77% H, 18.88% N. Found: 52.60% C, 5.97% H, 18.64% N.

### EXAMPLE 2

### Synthesis of N¹-(4-sec-Butylphenyl)-N²-(2-chloroethyl)-1,2-diazenedicarboxamide

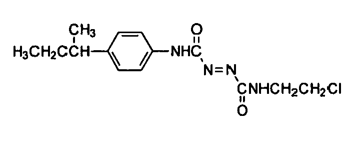

### Step 1

### Methyl (4-sec-Butylanilino)carbonylhydrazinecarboxylate

A solution of 4-sec-butylaniline (1.53 mL, 10 mmol) in dichloromethane (10 mL) was added dropwise to the solution of trifosgene (1.10 g, 3.7 mmol) in dichloromethane (10 mL) under argon at rt. A suspension was cooled to 0 °C and treated with triethylamine (3 ml, 23.3 mmol). After 5 min, a methyl hydrazinecarboxylate (900 mg, 10 mmol) was added and the reaction mixture was stirred 30 min at 0 °C, followed by 30 min at rt. Then, HCl (15 mL, 1M) was added to give white participation. The solid material was filtered off, washed successively with water (7 mL) and diethyl ether (2 × 5mL) to produce 2.14 (81%) of the desired product.
mp = 184-185.7 °C (ethanol)
IR (KBr) 3362, 3308, 3250, 2959, 1736, 1707, 1686, 1609, 1549, 1248 cm⁻¹.
¹H NMR (DMSO-*d*₆) δ 0.76 (t, *J* = 7.3 Hz, 3H), 1.16 (d, *J* = 7.0 Hz, 3H), 1.51 (qd, *J₁* = 7.3 Hz, *J₂* = 1.9 Hz, 2H), 2.51 (qt, *J₃* = 7.0 Hz, *J₂* = 1.9 Hz, 1H), 7.07 (m, 2H), 7.38 (m, 2H), 7.97 (broad s, 1H), 8.61 (s, 1H), 8.95 (broad s, 1H).
¹³C NMR (DMSO-d₆) δ 12.0, 21.8, 30.6, 45.5, 51.8, 118.6, 126.8, 137.4, 140.5, 155.6, 157.3. MS (EI) m/z (%) 265 (M⁺, 20), 175 (40), 146 (100), 90 (95).
HRMS: Calcd for C₁₃H₁₉N₃O₃: 265.1426. Found: 265.1433.
Anal. Calcd for C₁₃H₁₉N₃O₃ (M = 265.31 g/mol): 58.85% C, 7.22% H, 15.84% N. Found: 58.72% C, 7.42% H, 15.84% N.

### Step 2

### Methyl (4-sec-Butylanilino)carbonyldiazenecarboxylate

NBS (979 mg, 5.5 mmol) was slowly added to a stirred suspension of methyl (4-*sec*-butylanilino)carbonylhydrazinecarboxylate (1.33 g, 5 mmol) and pyridine (810 µl, 10 mmol) in dichloromethane (15 mL) at rt. After 1 h, the reaction mixture was treated with HCl (1 : 1, 10 mL). Organic phase was washed successively with 5% solution of Na₂S₂O₃ × 5H₂O (15 mL), saturated NaHCO₃ (7 mL), and water (10 mL), dried over anhydrous Na₂SO₄, and evaporated to dryness to give 1.01 g (77%) of an oily product.
mp = oily product
IR (NaCl) 3274, 2961, 1776, 1736, 1730, 1602, 1560, 1516, 1437, 1417, 1251 cm⁻¹.
¹H NMR (DMSO-d₆) δ 0.77 (t, *J* = 7.3 Hz, 3H), 1.18 (d, *J* = 7.0 Hz, 3H), 1.55 (m, 2H), 2.60 (m, 1H), 4.07 (s, 3H), 7.25 (m, 2H), 7.64 (m, 2H), 11.45 (s, 1H).
¹³C NMR (DMSO-*d₆*) δ 12.1, 21.7, 31.1, 41.2, 55.4, 119.7, 127.9, 133.6, 145.8, 155.0, 162.1.
MS (FAB) m/z (%) 264 (M⁺ + H, 41), 176 (100), 148 (85), 92 (65).
Molecular formula: C₁₃H₁₇N₃O₃ M = 263.29 g/mol

### Step 3

### N¹-(4-sec-Butylphenyl)-N²-(2-chloroethyl)-1,2-diazenedicarboxamide

To a stirred suspension of methyl (4-*sec*-butylanilino)carbonyldiazenecarboxylate (1.00 g, 3.8 mmol) and Na₂CO₃ (403 mg, 3.8 mmol) in ethyl acetate (15 mL), a 2-chloroethylamine hydrochloride (441 mg, 3.8 mmol) was added portionwise at 0 °C. After 4 hrs at this temperature, water was added (5 mL) and two phases were separated. An organic phase was dried over anhydrous Na₂SO₄ and then evaporated to dryness to afford 1.11 g (94%) of the final product.
mp = 115.8-119 °C (petroleum ether/ethyl acetate)
IR (KBr) 3246, 2965, 2928, 1735, 1708, 1601, 1532, 1416, 1317, 1308, 1247, 1190, 1059 cm⁻¹.
¹H NMR (DMSO-*d*₆) δ 0.77 (t, *J* = 7.3 Hz, 3H), 1.18 (d, *J* = 7.0 Hz, 3H), 1.55 (qd, *J₁* = *J₂* = 7.2 Hz, 2H), 2.59 (m, 1H), 3.63 (m, 2H), 3.78 (t, *J* = 5.9 Hz, 2H), 7.24 (m, 2H), 7.64 (m, 2H), 9.23 (broad t, *J* = 5.5 Hz, 1H), 11.27 (s, 1H).
¹³C NMR (DMSO-*d*₆) δ 12.0, 21.7, 30.5, 40.4, 42.1, 42.9, 119.6, 127.4, 135.2, 143.9, 158.1, 161.9.
MS (FAB) m/z (%) 313 ((M + 2)⁺ + H, 3), 185 (97), 93 (100).
Anal. Calcd for C₁₄H₁₉ClN₄O₂ (M = 310.78 g/mol): 54.11 % C, 6.16% H, 18.03% N. Found: 54.30% C, 5.96% H, 17.91 % N.

### EXAMPLE 3

### Synthesis of N¹-(4-tert-Butylphenyl)-N²-(2-chloroethyl)-1,2-diazenedicarboxamide

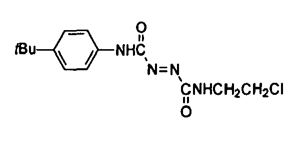

### Step 1

### Methyl (4-tert-Butylanilino)carbonylhydrazinecarboxylate

A solution of 4-tert-butylaniline (800 µl, 5 mmol) in dichloromethane (10 mL) was added dropwise to the solution of trifosgene (549 mg, 1.85 mmol) in dichloromethane (10 mL) under argon at rt. A suspension was cooled to 0 °C and treated with triethylamine (1.5 ml, 11.7 mmol). After 5 minutes, a methyl hydrazinecarboxylate (450 mg, 5 mmol) was added and the reaction mixture was stirred for 30 min at 0 °C, followed by 30 min at rt. Then, HCl (20 mL, 1M) was added to give white participation. The solid material was filtered off, washed successively with water (7 mL) and diethyl ether (3 × 5mL) to produce 1.20 (91%) of the desired product.
mp = 185-187 °C (dichloromethane/methanol)
IR (KBr) 3304, 2959, 1731, 1686, 1609, 1553, 1314, 1259cm⁻¹.
¹H NMR (DMSO-*d*₆) δ 1.25 (s, 9H), 3.61 (s, 3H), 7.25 (m, 2H), 7.37 (m, 2H), 7.96 (broad s, 1H), 8.62 (s, 1H), 8.94 (broad s, 1H).
¹³C NMR (DMSO-*d*₆) δ 31.2, 33.8, 51.9, 118.3, 125.1, 137.0, 144.1, 155.6, 157.4.
MS (EI) m/z (%) 265 (M⁺, 20), 175 (32), 160 (100), 90 (95).
HRMS Calcd for C₁₃H₁₉N₃O₃: 265.1426. Found: 265.1431.
Anal. Calcd for C₁₃H₁₉N₃O₃ (M = 265.31 g/mol): 58.85% C, 7.22% H, 15.84% N. Found: 59.13% C, 7.47% H, 16.03% N.

### Step 2

### Methyl (4-tert-Butylanilino)carbonyldiazenecarboxylate

NBS (783 mg, 4.4 mmol) was slowly added to a stirred suspension of methyl (4-tert-butylanilino)carbonylhydrazinecarboxylate (1.06 g, 4 mmol) and pyridine (645 µl, 8 mmol) in dichloromethane (10 mL) at rt. After 30 min, the reaction mixture was treated with HCl (1 : 1, 10 mL). An organic phase was washed successively with 5% solution of Na₂S₂O₃.5H₂O (3 mL), saturated NaHCO₃ (10 mL), and water (10 mL), dried over anhydrous Na₂SO₄, and evaporated to dryness to achieve 963 mg (92%) of an oily product.
mp = oily product
IR(NaCl) 3270, 2960, 1773, 1730, 1601, 1560, 1522, 1437, 1406, 1252 cm⁻¹.
¹H NMR (DMSO-*d*₆) δ 1.28 (s, 9H), 4.07 (s, 3H), 7.44 (m, 2H), 7.65 (m, 2H), 11.45 (s, 1H).
¹³C NMR (DMSO-*d*₆) δ 31.0, 34.1, 55.6, 119.4, 125.8, 134.6, 147.6, 157.0, 161.8.
Molecular formula: C₁₃H₁₇N₃O₃ M = 263.29 g/mol

### Step 3

### N¹-(4-tert-Butylphenyl)-N²-(2-chloroethyl)-1,2-diazenedicarboxamide

To a stirred suspension of methyl (4-tert-butylanilino)carbonyldiazenecarboxylate (920.5 mg, 3.5 mmol) and Na₂CO₃ (371 mg, 3.5 mmol) in dichloromethane (5 mL), a 2-chloroethylamine hydrochloride (406 mg, 3.5 mmol) was added portionwise at 0 °C. After 2 hrs at 0 °C, the reaction mixture was evaporated to dryness, the residue was treated with ethyl acetate (15 mL) and a solution was washed with water (2 × 10 mL). Organic phase was dried over anhydrous Na₂SO₄ and ethyl acetate was removed under reduced pressure to give 980 mg (90%) of the final product.
mp = 125.9-127.7 °C (ethyl acetate/petroleum ether)
IR (KBr) 3236, 3040, 2962, 1735, 1699, 1536, 1518, 1365, 1267, 835 cm⁻¹.
¹H NMR (DMSO-d₆) δ 1.28 (s, 9H), 3.63 (m, 2H), 3.78 (t, *J* = 5.9 Hz, 2H), 7.43 (m, 2H), 7.64 (m, 2H), 9.23 (broad t, *J* = 5.6 Hz, 1H).
¹³C NMR (DMSO-d₆) δ 31.1, 34.1, 42.1, 42.9, 119.4, 125.7, 134.9, 147.4, 158.1, 161.9.
MS (FAB) m/z (%) 313 ((M + 2)⁺ + H, 23).
Anal. Calcd for C₁₄H₁₉ClN₄O₂ (M = 310.78 g/mol): 54.11% C, 6.16% H, 18.03% N. Found: 54.39% C, 6.42% H, 17.97% N.

### EXAMPLE 4

### Synthesis of N¹-(2-Chloroethyl)-N²-(2-pyridylmethyl)-1,2-diazenedicarboxamide

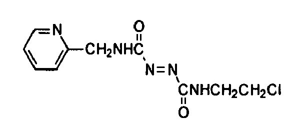

Methyl (2-chloroethylamino)carbonyldiazenecarboxylate (290.3 mg, 1.5 mmol; prepared according to Bombek, S.; Pozgan, F.; Kocevar, M.; Polanc, S., J. Org. Chem. 2004, 69, 2224-2227.) was added portionwise to a stirred solution of 2-picolylamine (155 µl, 1.5 mmol) v acetonitrile (3 mL) at 0 °C. After 45 min at 0 °C, the solid material was filtered off and washed with cold acetonitrile (1 mL) to produce 337.9 mg (83%) of diazenedicarboxamide.
mp = 142.6-144.1 °C (ethyl acetate/methanol)
IR (KBr) 3247, 1746, 1712, 1594, 1537, 1474, 1437, 1360, 1265, 1225, 1192, 1063 cm⁻¹.
¹H NMR (DMSO-*d₆*) δ 3.60 (m, 2H), 3.77 (t, *J* = 5.9 Hz, 2H), 4.55 (d, *J* = 6.0 Hz, 2H), 7.30 (ddd, *J₁* = 7.5 Hz, *J₂* = 4.9 Hz, J₃ = 1.1 Hz, 1H), 7.38 (ddd, *J₁* = 7.7 Hz, *J₂* = 1.1 Hz, *J₃ =* 0.9 Hz, 1 H), 7.80 (ddd, *J₁* = 7.7 Hz, *J₂* = 7.5 Hz, *J₃* = 1.9 Hz, 1 H), 8.54 (ddd, *J₁* = 4.9 Hz, *J₂* = 1.9 Hz*, J₃* = 0.9 Hz, 1H), 9.09 (broad t, *J* = 5.7 Hz, 1H), 9.41 (broad t, *J* = 6.0 Hz, 1H).
¹³C NMR (DMSO-*d₆*) δ 42.0, 43.0, 45.3, 121.3, 122.5, 136.9, 149.1, 157.1, 161.8, 162.0.
MS (FAB) *m*/*z* (%) 270 (M⁺ + H, 43), 154 (51), 13 (100).
Anal. Calcd for C₁₀H₁₂ClN₅O₂ (M = 269.69 g/mol): 44.54% C, 4.48% H, 25.97% N. Found: 44.43% C, 4.53% H, 26.03% N.

### EXAMPLE 5

### Synthesis of N¹-Phenyl-N²-(3-picolyl)diazenedicarboxamide

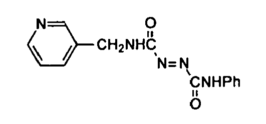

Ethyl phenylaminocarbonyldiazenecarboxylate (1.327 g, 6 mmol; prepared according to Knight, G. T.; Loadman, J. R.; Saville, B.; Wildgoose, J., J. Chem. Soc., Chem. Commun. 1974, 193-194.) was added portionwise to a stirred solution of 3-picolylamine (0.610 ml, 6 mmol) in acetonitrile (3 mL) at 0 °C. After 15 min at 0 °C, the solid material was filtered off and washed with cold acetonitrile to achieve 1.472 g (87%) of the product.
mp = 156-157 °C (ethyl acetate).
IR (KBr) 3300, 2940, 1710, 1590, 1515, 1440, 1315, 1250, 1030 cm⁻¹.
¹H NMR (DMSO-d₆) δ 4.53 (d, 2H, *J* = 5.9 Hz), 7.19 (m, 1H), 7.41 (m, 3H), 7.72 (m, 2H), 7.77 (ddd, 1H, *J*₁ = 7.8 Hz, *J*₂ = 2.3 Hz, *J*₃ = 1.6 Hz), 8.51 (dd, 1 H, *J*₁ = 4.8 Hz, *J*₂ = 1.6 Hz), 8.59 (dd, 1H, *J*₁ = 2.3 Hz, J₂ = 0.8 Hz), 9.57 (t, 1 H, *J* = 5.9 Hz), 11.31 (s, 1 H).
¹³C NMR (DMSO-d₆) δ 41.2, 119.6, 123.6, 124.9, 129.1, 133.6, 135.4, 137.4, 148.6, 148.9, 158.2, 161.9.
MS (FAB) m/z (%) 284 (M⁺ + H, 30), 120 (14), 107 (25).
Anal. Calcd for C₁₄H₁₃N₅O₂ (M = 283.29 g/mol): 59.36% C, 4.63% H, 24.72% N. Found: 59.64% C, 4.53% H, 24.88% N.

### Example 6

### Synthesis of N¹-(3-Chlorophenyl)-N²-(3-picolyl)diazenedicarboxamide

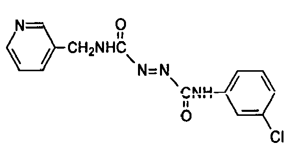

### Step 1

### Ethyl (3-Chlorophenyl)aminocarbonyldiazenecarboxylate

NBS (2.723 g, 15.3 mmol) was added portionwise to a stirred suspension of ethyl (3-chlorophenyl)aminocarbonylhydrazinecarboxylate (3.865 g, 15 mmol; prepared according to Bollbuck, G.; Stroh, H-H. Barnikow, G., J. Prakt. Chem. 1971, 313, 773-777.) and pyridine (2.43 mL, 30 mmol) in dichloromethane (60 mL) at rt. After 45 min, the reaction mixture was treated with HCl (1:1, 40 mL), two phases were separated and an organic one was washed successively with aqueous solution of Na₂S₂O₃.5H₂O (1.5 g, 60 mL), saturated solution of NaHCO₃ (60 mL) and water (60 mL). An organic phase was dried over anhydrous Na₂SO₄ and the solvent was removed under reduced pressure to afford 3.731 g (97%) of the desired diazene-carboxylate.
mp = 49.5-50.5 °C (cyclohexane/toluene).
IR (KBr) 3300, 3000, 1780, 1740, 1600, 1490, 1250, 1030 cm⁻¹.
¹H NMR (DMSO-*d*₆) δ 1.38 (t, 3H, *J* = 7.2 Hz), 4.53 (q, 2H, *J* = 7.2 Hz), 7.29 (ddd, 1H, *J*₁ = 8.0 Hz, *J*₂ = 2.1 Hz, *J*₃ = 1.0 Hz), 7.46 (dd, 1H, *J*₁ = 8.0 Hz, *J*₂ = 8.2 Hz), 7.66 (ddd, 1H, *J*₁ = 8.2 Hz, *J*₂ = 2.0 Hz, *J*₃ = 1.0 Hz), 7.85 (dd, 1H, *J*₁ = 2.1 Hz, *J*₂ = 2.0 Hz), 11.71 (s, 1H).
¹³C NMR (DMSO-*d*₆) δ 13.9, 65.5, 118.2, 119.2, 124.9, 130.8, 133.4, 138.7, 157.0, 161.2.
MS (FAB) m/z 256 (M⁺ + H, 16), 167 (9), 107 (25).
Anal. Calcd for C₁₀H₁₀ClN₃O₃ (M = 255.66 g/mol): 46.98% C, 3.94% H, 16.44% N. Found: 47.20% C, 3.97% H, 16.36% N.

### Step 2

### N¹-(3-Chlorophenyl)-N²-(3-picolyl)diazenedicarboxamide

Ethyl (3-chlorophenyl)aminocarbonyldiazenecarboxamide (1.534 g, 6 mmol) was added portionwise to a stirred solution of 3-picolylamine (0.610 mL, 6 mmol) in acetonitrile (2 mL) at 0 °C. After 15 min, a yellow solid was filtered off and washed with acetonitrile to give 1.412 g (74%) of the final product.
mp = 129-130 °C (ethyl acetate).
IR (KBr) 3338, 2939, 1741, 1713, 1546, 1504, 1428, 1189 cm⁻¹.
¹H NMR (DMSO-*d*₆) δ 4.54 (d, 2H, *J* = 6.0 Hz), 7.27 (ddd, 1H, *J*₁ = 8.0 Hz, *J*₂ = 2.1 Hz, *J*₃ = 1.0 Hz), 7.41 (ddd, 1H, *J*₁ = 7.8 Hz, *J*₂ = 4.8 Hz, *J*₃ = 0.9 Hz), 7.45 (dd, 1H, *J*₁ = 8.2 Hz, *J*₂ = 8.0 Hz), 7.66 (ddd, 1H, *J*₁ = 8.2 Hz, *J*₂ = 2.0 Hz, *J*₃ = 1.0 Hz), 7.78 (ddd, 1H, *J*₁ = 7.8 Hz, *J*₂ = 2.4 Hz, *J*₃ = 1.7 Hz), 7.86 (dd, 1H, *J*₁ = 2.1 Hz, *J*₂ = 2.0 Hz), 8.52 (dd, 1H, *J*₁ = 4.8 Hz, *J*₂ = 1.7 Hz), 8.60 (dd, 1H, *J*₁ = 2.4 Hz, *J*₂ = 0.9 Hz), 9.63 (t, 1H, J= 6.0 Hz), 11.55 (s, 1H).
¹³C NMR (DMSO-*d*₆) δ 41.2, 118.1, 119.0, 123.6, 124.7, 130.9, 133.4, 133.6, 135.4, 138.9, 148.6, 148.9, 158.2, 161.7.
MS (FAB) m/z (%) 318 (M⁺ + H, 30), 123 (22), 107 (33).
Anal. Calcd for C₁₄H₁₂ClN₅O₂ (M = 317.73 g/mol): 52.92% C, 3.81% H, 22.04% N. Found: 53.14% C, 3.88% H, 21.72% N.

### Example 7

Inhibitory and antimicrobial activities of some compounds.

| Structure | DdlB | MIC | | |
|---|---|---|---|---|
| | | *E.coli* 1411 | *E.coli* SM 1411 | *S.aureus* 8325-4 |
| 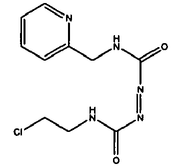 | **IC₅₀=119µM** | >256 | >256 | >256 |
| 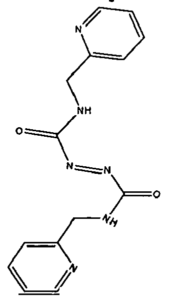 | **IC₅₀=158µM** | >256 | >256 | >256 |
| 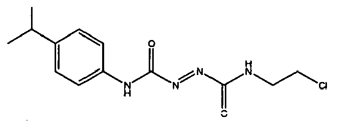 | **IC₅₀=49µM** | >256 | >256 | >256 |
| 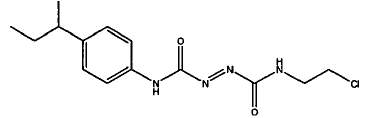 | **IC₅₀=25µM** | >256 | >256 | >256 |
| 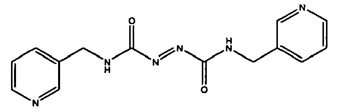 | **IC₅₀=123µM** | >256 | >256 | >256 |
| 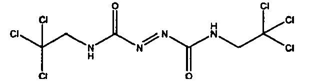 | **IC₅₀=187µM** | >256 | >256 | >256 |
| 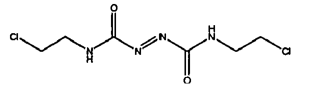 | **IC₅₀=133µM** | >256 | >256 | >256 |
| 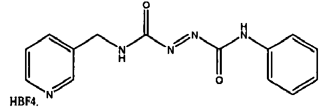 | **IC₅₀=76µM** | >256 | >256 | >256 |
| 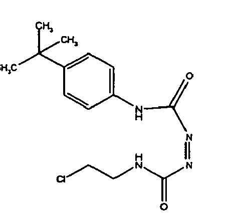 | **IC₅₀=37µM** | >256 | >256 | >256 |
| 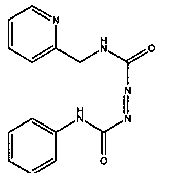 | **IC₅₀=111µM** | 64 | 64 | 64 |
| 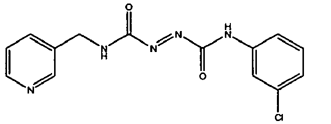 | **IC₅₀=15µM** | 64 | 64 | 256 |
| 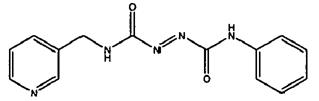 | **IC₅₀=36µM** | 64 | 64 | 128 |
| 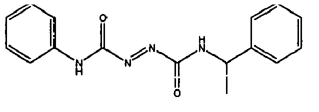 | **IC₅₀=41µM** | >256 | >256 | >256 |

## Claims

1. Compounds with general formula I, 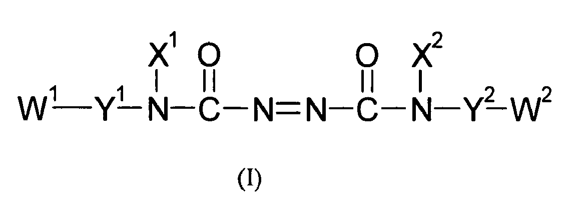 wherein:
**X¹, X²**: H, C₁₋₆ linear or branched alkyl, (aryl) -NH- , -O-, -(CH₂)ₙ- (where n can be between 0 and 5), C₁₋₆ branched alkyl;
**Y¹, Y²**: -(CH₂)ₙ- (where n can be between 0 and 5), C₁₋₆ branched alkyl; -NH-, -C=O; -NHCO-, -CONH-;
**W¹, W²**: - H,
- C₁₋₆ linear or branched alkyl. Alkyl can either be unsubstituted or substituted with one or more substituents, e.g., nitro, halogen, trifluoromethyl or hydroxyl,
- phenyl, which can either be unsubtsituted or substituted with one or more substituents, e.g., nitro, halogen, trifluoromethyl, hydroxyl, C₁₋₈ linear or branched alkyl, C₁₋₈ linear or branched alkoxy, unsubstituted phenyl or substituted phenyl,
- naphthyl, which can be either unsubsituted or substituted
- fluorenyl,
- 5- to 7-membered monocyclic or 7- to 10-membered bicyclic heterocyclic ring system, which can be unsubstituted or substituted and can beside carbon atoms contain 3 or less heteroatoms such as N, O, P or S,
- 3- to 10- membered monocyclic or 7- to 10- membered carbocyclic ring system, which can be unsubstituted or substituted and can beside carbon atoms contain 3 or less heteroatoms such as N, O, P or S.

2. Compounds with general formula I of Claim 1 for use as therapeutically active substances.

3. A process for the preparation of the compounds having the formula I of Claim 1 **characterised in that**:
a) unsymmetrical derivative is synthesized from isocyanate with formula II (prepared from corresponding amine with phosgene, diphosgene or triphosgene in dry aprotic solvent in the presence of tertiary amine) and carbazate III (R = C₁₋₆ linear or branched alkyl) in aprotic solvent, 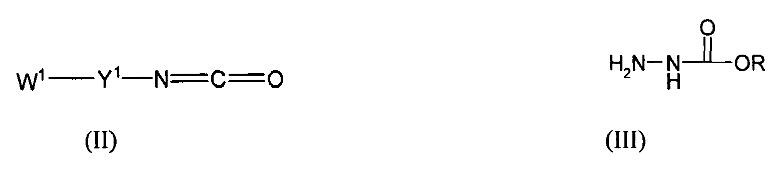 this results in corresponding 1,4-disubstituted semicarbazide IV; the same compound can be prepared from 4-substituted semicarbazide V and chloroformiate VI (R = C₁₋₆ linear or branched alkyl), if semicarbazide V reacts with chloroformiate VI, 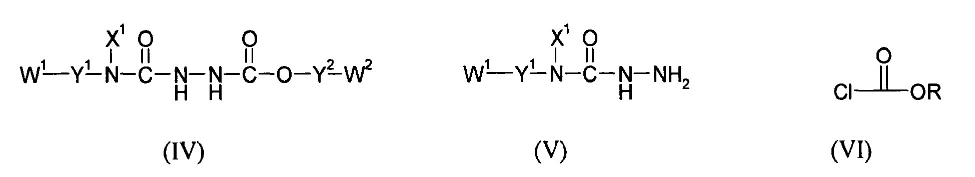 resulted 1,4-disubstituted semicarbazide IV is transformed to aminocarbonyldiazenecarboxylate VII with suitable organic or inorganic oxidizing agent; substitution of alkoxy group of aminocarbonyldiazenecarboxylate VII with primary or secondary amines VIII results in diazenedicarboxamide I; 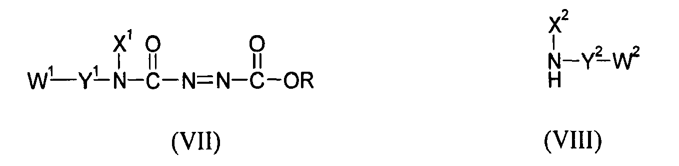
b) symmetrical diazenedicarboxamide derivative I (X¹ = X², Y¹ = Y², W¹ = W²) can be prepared from diazenedicarboxylate and two or more equivalents of primary or secondary amine in protic or aprotic solvent; nucleophilic substitution of both alkoxy groups can be also carried out in the absence of solvent.

4. The use of compounds with general formula I of Claim 1 as therapeutically active substances for drug preparation; these compounds are inhibitors of D-alanine:D-alanine ligase and can be used for treatment of bacterial infections.

5. Pharmaceutical compositions **characterised in that** they are comprising a therapeutically effective amount of the compound having the formula I of Claim 1 and pharmaceutically acceptable auxiliary substances.

6. The pharmaceutical compositions of Claim 5 **characterised in that** they are used against microbial infections in man or other mammals.
